Europäisches Patentamt

European Patent Office

Office européen des brevets

(19)

(11) Publication number: **0 375 127**
**A1**

(12) ## EUROPEAN PATENT APPLICATION

(21) Application number: **89311304.3**

(22) Date of filing: **01.11.89**

(51) Int. Cl.5· **A61K 37/02, A61L 15/16,**
**A61L 15/32**

(30) Priority: **02.11.88 US 266179**

(43) Date of publication of application:
**27.06.90 Bulletin 90/26**

(84) Designated Contracting States:
**AT BE CH DE ES FR GB GR IT LI LU NL SE**

(71) Applicant: **GENENTECH, INC.**
**460 Point San Bruno Boulevard**
**South San Francisco California 94080(US)**

(72) Inventor: **Ammann, Arthur S.**
**104 Dominican Drive**
**San Rafael California 94901(US)**
Inventor: **Snyderman, Ralph**
**P.O. Box 3701**
**Durham, NC 27710(US)**

(74) Representative: **Armitage, Ian Michael et al**
**MEWBURN ELLIS & CO. 2/3 Cursitor Street**
**London EC4A 1BQ(GB)**

(54) **Composition for the treatment of periodontal disease.**

(57) A method is provided for the treatment of periodontal disease involving administering to a mammal suffering from gum disease a physiologically effective amount of transforming growth factor-$\beta$ (TGF-$\beta$) formulated in a suitable composition. The mammal is preferably human. Also provided is a permeable, dissolvable, therapeutic material that is treated with TGF-$\beta$ and is shaped or flexed to fit around the teeth or gums.

EP 0 375 127 A1

# COMPOSITION FOR THE TREATMENT OF PERIODONTAL DISEASE

This invention relates to the treatment of periodontal disease using a composition of aTGF-β.

In the development of periodontal disease, bacterial plaque and calculus accumulate on the teeth, followed by the clinical signs of acute gingivitis. Gingivitis is an inflammatory process affecting the soft tissues surrounding the teeth. While plaque-associated gingivitis is the most common form, other forms exist such as acute necrotizing ulcerative gingivitis, juvenile periodontal disease, steroid-hormone-influenced gingivitis, medication-influenced gingival overgrowth (e.g., by phenytoin or cyclosporin A), and other forms, such as those induced by nutritional deprivation. Gingivitis is diagnosed when the gingiva appears red and puffy, loses its stippling, and bleeds spontaneously or on probing. Plaque-induced gingivitis is reversible by scaling and cleaning the teeth.

If the bacterial plaque is not removed, the gingivitis progresses through the stages of chronic gingivitis and chronic periodontitis to chronic destructive periodontitis. Most forms of periodontitis in adults are categorized into a single class, chronic adult periodontitis, the pathogenesis of which is localized chronic inflammation with concomitant connective tissue and bony degeneration. The terminal stages of this disease are characterized by mobility, drifting and tilting of the teeth, loss of epithelial attachment, collagen destruction, periodontal pocket formation, and massive loss of supporting alveolar bone. Additional categories of periodontitis mostly represent forms of the disease occurring in younger patients, including rapidly progressive periodontitis, juvenile periodontitis, postjuvenile periodontitis, and prepubertal periodontitis.

The native connective tissue attachment of the periodontium is known to be a complex consisting of fibroblasts (including gingival fibroblasts and periodontal ligament cells), epithelial cells, vascular endothelial cells, nerve cell processes, cementum, alveolar bone, and extensive extracellular matrix (collagen, glycoproteins, and proteoglycans). Thus, excess collagenolytic activity may be manifested by periodontitis. The regeneration of this complex following periodontal disease is one ultimate goal of clinical periodontal therapy.

Another goal is to eliminate the plaque formed in locations of the oral cavity with access to the gingival sulcus. Dentogingival plaque represents an aggregate of diverse constituents concentrated in a matrix that adheres to the oral tissues. The constituents include immunopotentiating and immunosuppressive agents such as lipopolysaccharides, dextrans, and levans; cariogenic microorganisms such as Streptococcus mutans, Actinomyces viscosus, and lactobacilli; periodontal microorganisms such as Actinomyces, Actinobacillus, Veillonella, Bacteroides, Eikenella, Capnocytophaga, Wolinella, Fusobacterium, Treponema, and Spirochaeta; proteoglycans;glycoproteins; phospholipids; immunoglobulins; desquamated epithelial cells; and polymorphonuclear cells. This matrix enables the plaque to produce sufficient amounts of bacterial end products to cause and sustain the inflammatory condition, and, in time, to damage the soft tissues as is characteristic of gingivitis and periodontitis.

Routine daily prevention or removal of plaque by the patient is essential in treatment of periodontitis. Such mechanical removal, using toothbrushes, floss or other oral hygiene instruments, however, requires that the patient be dexterous and motivated to remove the plaque. Dental instruments also are employed to remove plaque and calculus mechanically from the root surface. In addition, subgingival scaling and root planning, gingival curettage, and surgery, such as removal of periodontal tissues using a modified Widman flap procedure, have been employed.

Topical preparations such as ointments, dentifrices, gums, and mouth rinses also have been employed to treat gingivitis. Such agents include antibiotics, such as tetracycline, enzyme preparations such as dehydrated pancreas preparations, phenolic compounds as in mouth rinses, herbal extracts with benzophenathridine alkaloid mixture, such as sanguinarine, quaternary ammonium compounds that are surface active agents, fluorides, and bisbiguanides. However, indigenous bacteria may develop resistance to the antibiotics. Also, such preparations are effective only on supragingival bacteria, not on the subgingival plaque associated with periodontitis.

For removal of subgingival plaque, a chemotherapeutic agent is administered for extended periods of time systemically or at the site of action, e.g., in a periodontal pocket. Examples of agents used include non-steroidal anti-inflammatory agents such as flurbiprofen, antibiotics such as minocycline, and metronidazole. Many of these drugs have side effects or induce resistance to antibiotics.

Another approach has been to develop a vaccine composed of antibodies directed against antigenic determinants of A. viscosus fimbriae to inhibit their attachment to the teeth.

Citric acid conditioning or insertion of synthetic membranes, allowing for selective cell recolonization of the tooth root surface by cells of mesenchymal origin, has been used as an adjunct to periodontal surgery. In addition, tetracycline HCl has been shown to demineralize root surface dentin, adsorb to enamel and dentin, and inhibit collagenolytic enzyme activity and bone resorption. Further, when the dentin surface is preconditioned with tetracycline HCl and treated with fibronectin and endothelial cell growth factor, periodontal ligament cells exhibit enhanced migration to and proliferation on the dentin surface. Terranova et al., J. Periodontol., 58: 247 (1987). Also, laminin has been shown to enhance gingival epithelial cell chemotaxis, proliferation and movement. Terranova et al., supra; Terranova et al., J. Periodontol., 57: 311 (1986); Terranova et al., J. Periodont. Res., 21: 330 (1986).

It has also been suggested that tetracycline HCl and/or citric acid preconditioning of the root surface and subsequent application of biological response modifiers, such as polypeptide growth factors, may promote a connective tissue healing at the dentin-soft tissue interface. Terranova et al., J. Periodontol., 58 371-380 (1987).

The transforming growth factor-$\beta$ (TGF-$\beta$) molecules identified thus far are each dimers containing two identical 112 residue polypeptide chains linked by disulfide bonds. The molecular mass of these dimers is about 25 kd. Biologically active TGF-$\beta$ has been defined as a molecule capable of inducing anchorage independent growth of target cell lines or rat fibroblasts in in vitro cell culture, when added together with EGF or TGF-$\alpha$ as a cofactor. TGF-$\beta$ is secreted by virtually all cell types in an inactive form. This latent form is first activated by proteolytic cleavage of mature TGF-$\beta$ from its precursor (at the Arg-Ala bond in position 278). A non-covalent complex is formed from the association of the mature TGF-$\beta$ with the precursor remainder or with a protein binding to TGF-$\beta$ or with alpha$_2$-macroglobulin. This complex is disrupted so as to activate the TGF-$\beta$ either by exposure to transient acidification or by the action of exogenous proteases.

There are at least three forms of TGF-$\beta$ currently identified, TGF-$\beta_1$, TGF-$\beta_2$, and TGF-$\beta_3$. Suitable methods are known for purifying this family of TGF-$\beta$s from platelets or placenta, for producing it in recombinant cell culture, and for determining its activity. See, for example, R. Derynck et al., Nature, 316:701 (1985) European Pub. No. 128,849 and PCT Pub. No. WO84/01106; European Pat. Pub. Nos. 200,341 published December 10,1986, 169,016 published January 22, 1986; 268,561 published May 25, 1988; and 267,463 published May 18, 1988; U.S. Pat. No. 4,774,322; Seyedin et al., J. Biol. Chem., 262: 1946-1949 (1987); Cheifetz et al., Cell, 48: 409-415 (1987);

Jakowlew et al., Molecular Endocrin., 2:747-755 (1988); and Dijke et al., Proc. Natl. Acad. Sci. U.S.A., 85: 4715-4719 (1988).

TGF-$\beta$ has been shown to have numerous regulatory actions on a wide variety of both normal and neoplastic cells. Recent studies indicate an important role for TGF-0 in cells of the immune system (J. Kehrl et al., J. Exp. Med., 163: 1037 [1986]; H-J. Ristow, Proc. Natl. Acad. Sci. U.S.A., 83:5531 [1986]; A. Rook et al., J. Immunol., 136:3916[1986]) and in proliferation of connective and soft tissue for wound healing applications (M. Sporn et al., Science, 219:1329 [1983]; R. Ignotz et al., J. Biol. Chem., 261:4337 [1986]; J. Varga et al., B.B.Res.Comm., 138:974 [1986]; A. Roberts et al., Proc. Natl. Acad. Sci. U.S.A., 78:5339 [1981]; A. Roberts et al., Fed. Proc., 42:2621 [1983]; A. Roberts et al., Proc. Natl. Acad. Sci. U.S.A., 83:4167 [1986]; PCT Pub. No. WO84/01106, supra; U.S. Pat. No. 4,774,228 to Seyedin et al.), as well as epithelia (T. Matsui et al., Proc. Natl. Acad. Sci. U.S.A., 83:2438 [1986]; G. Shipley et al. Cancer Res., 46:2068 [1986]). Moreover, TGF-$\beta$ has been described as a suppressor of cytokine (e.g., IFN-$\gamma$, TNF-$\alpha$) production, indicating its use as an immunosuppressant for treating inflammatory disorders (Espevik et al., J. Exp. Med., 166: 571-576 [1987]; European Pat. Pub. Nos. 269,408 published June 1, 1988 and 213,776 published March 11, 1987), and as a promoter of cachexia (Beutler and Cerami, New Eng. J. Med., 316: 379 [1987]). Further, TGF-$\beta$ induces collagenase secretion in human fibroblast cultures (Chua et al., J. Biol. Chem., 260:5213-5216 [1983]); stimulates the release of prostaglandins and mobilization of calcium (A. Tashjian et al., Proc. Natl. Acad. Sci. U.S.A., 82:4535 [1985]); and inhibits endothelial regeneration (R. Heimark et al., Science, 233:1078 [1986]).

TGF-$\beta$ is multifunctional, as it can either stimulate or inhibit cell proliferation, differentiation, and other critical processes in cell function (M. Sporn, Science, 233:532 [1986]).

The multifunctional activity of TGF-$\beta$ is modulated by the influence of other growth factors present together with the TGF-$\beta$. TGF-$\beta$ can function as either an inhibitor or an enhancer of anchorage-independent growth, depending on the particular set of growth factors, e.g., EGF or TGF-$\alpha$, operant in the cell together with TGF-$\beta$ (Roberts et al., Proc. Natl. Acad. Sci. US.A., 82:119 [1985]). TGF-$\beta$ also can act in concert with EGF to cause proliferation and piling up of normal (but not rheumatoid) synovial cells (Brinkerhoff et al., Arthritis and Rheumatism, 26:1370 [1983]).

Most recently, TGF-$\beta$ has been found to suppress the expression of Class II histocompatibility antigens on human cells induced by human interferon-$\gamma$ (Czarniecki et al., J. Immunol.,

140:4217-42223 [1988]; Czarniecki et al. J. Interferon Res., 7:699 [1987]; Palladino et al., J. Cell. Biochem., Supp. 11A [Jan. 17-Feb. 5, 1987], UCLA Symposia on Molecular and Cellular Biology, Alan R. Liss, Inc., New York, abstract A016, p. 10; Chiu et al., Triennial Symposium: Biology of Growth Factors, University of Toronto, Ontario, Canada, [June 17-19, 1987]; Palladino et al., Immunobiology, 175: 42 [1987]).

For a general review of TGF-$\beta$ and its actions, see Sporn et al., J. Cell Biol., 105: 1039-1045 (1987) and Sporn and Roberts, Nature, 332: 217-219 (1988).

There is a need in the art for dental compositions for the treatment of diseases of the gum that do not incur resistance to antibiotics.

In one aspect of the present invention there is provided a dental composition for use in treatment or prophylaxis of diseases of the oral cavity, including gingivitis and periodontitis by administration to the gum of a mammal a therapeutically effective amount of a composition comprising transforming growth factor-$\beta$(TGF-$\beta$) in a pharmaceutically acceptable carrier.

In another aspect, there is provided a permeable material that is shaped or flexes to fit around the teeth or gums of a mammal, which material is treated with TGF-$\beta$ and is capable of dissolving upon placement in an oral environment.

The term "diseased gum" as used herein refers to gum tissue that exhibits the pathologic symptoms associated with diseases of the oral cavity, including gingivitis and periodontitis in any of their forms, whether acute or chronic. Such symptoms include, but are not limited to, redness and puffiness of the gingiva, loss of stippling of the gingiva, bleeding, probing, mobility, drifting and tilting of the teeth, loss of epithelial attachment, collagen destruction, periodontal pocket formation, and loss of supporting alveolar bone.

The term "TGF-$\beta$" refers to the family of molecules described hereinabove. Reference to TGF-$\beta$ herein will be understood to be a reference to any one of the three currently identified forms, TGF-$\beta_1$, TGF-$\beta_2$ and TGF-$\beta_3$, as well as others identified in the future, their alleles, and their predetermined amino acid sequence variants, so long as they are effective in the method described herein. In addition, the TGF-$\beta$ is suitably useful in the latent form or as an associated or unassociated complex of precursor and mature TGF-$\beta$.

The TGF-$\beta$ is appropriately from any source, preferably mammalian, and most preferably human. TGF-$\beta$ from animals other than humans, for example, porcine or bovine sources, can be used for treating humans. Likewise, if it is desirable to treat other mammalian species such as domestic, farm, sports, or pet animals, human TGF-$\beta$, as well

as TGF-$\beta$ from other species, is suitably employed.

The term "chemotherapeutic agent" as used herein refers to substances other than TGF-$\beta$ that act as a therapeutic or prophylactic in the treatment of diseased gum. Examples of such agents include anti-microbial agents such as a tetracycline, especially minocycline; immunosuppressive or anti-inflammatory agents, e.g., non-steroidal anti-inflammatory drugs such as etodolac, glucosamine, D-mannosamine, D-galactosamine, mannose, cysteine, valine, alanine, and flurbiprofen, salicylate, penicillamine, gold salts, TNF-$\alpha$ or TNF-$\beta$ antagonists, such as anti-tumor necrosis factor-$\alpha$ antibodies and anti-tumor necrosis factor-$\beta$ antibodies, $\gamma$-interferon antagonists such as anti-interferon-$\gamma$ antibodies. IL-1 antagonists such as anti-IL-1 antibodies, azathioprine, or cyclosporin A; an alkaloid such as sanguinarine, which is an herbal extract containing a benzophenathridine alkaloid mixture; citric acid; a bisbiguanide such as chlorhexidine gluconate; a quaternary ammonium surface-active agent such as cetylpyridinium chloride or benzalkonium chloride; a fructan-degrading enzyme (U.S. Pat. No. 4,159,317); tolerstat; a peroxide; a fluoride such as stannous fluoride; a phenol such as thymol and/or eucalyptol; or a mixture thereof.

The preferred chemotherapeutic agent will depend on many factors, including the type of diseased gum being treated, as well as the patient's history, but a general overall preference is that the chemotherapeutic agent be selected from a tetracycline, cyclosporin A, sanguinarine, flurbiprofen, etodolac, metronidazole, chlorhexidine gluconate, cetylpyridinium chloride, benzalkonium chloride, stannous fluoride, thymol, eucalyptol, or a mixture of these agents.

As used herein, the expression "permeable material that is shaped or flexes to fit around the teeth or gums of a mammal" refers to a solid or semi-solid substance through which TGF-$\beta$ can permeate that can be shaped to provide a good fit around teeth or gums so as to be effective in delivering the TGF-$\beta$ to the site of interest. The size of the substance ranges from covering one small area to covering a large section of teeth or gum surface. This material also is capable of dissolving upon placement in an oral environment and releasing the TGF-$\beta$ as it dissolves. The material is suitably a membrane, strip, e.g., an acrylic resin strip, film, e.g., cast film of ethyl cellulose alone or with propylene glycol, fibers, e.g., permeable hollow cellulose acetate fibers filled with a solution of TGF-$\beta$ or solid absorbable fibers of polyglycolic acid incorporating TGF-$\beta$, a sheet, a bandage, a plastic hardenable mass, a medicated periodontal dressing, a microencapsulated liquid droplet formulation for topical application to the gums, a foam-film device, a dental flow, a dental adhesive such as guar gum or a cellulosic such as hydroxypropylmethyl cellulose, or a polymeric material or matrix, for example, a matrix composed of collagen, glycolic acid polymers, methacrylate polymers, or polylactides.

Also contemplated herein under this definition are substances with a putty-like consistency at room and body temperatures that can be conveniently shaped by hand into the form most desirable for placement in a periodontal pocket from which sustained release is desirable, as described by U.S. Pat. No. 4,568,536, where the preferred composition is a matrix of calcium stearate, dextran, and castor oil.

"Treatment" of the material with TGF-$\beta$ signifies surface treatment, absorption, impregnation, or other means to apply TGF-$\beta$ to the material in such a way that it can be effectively released when needed.

A dental composition comprising TGF-$\beta$ is administered to the gum of the mammal, preferably human. The administration may be prophylactic or therapeutic, i.e. to prevent, or prevent the worsening of, as well as alleviate, the pathogenic condition. The treatment suitably involves administering a therapeutically effective amount of the TGF-$\beta$ composition to the patient, preferably at the site of the diseased gum.

The TGF-$\beta$ compositions to be used in the therapy will be dosed in a fashion consistent with good medical practice taking into account the nature of the disorder to be treated, the species of the host, the medical condition of the individual patient, the presence of any other cotreatment drug in the composition, the site of delivery of the agent, the method of administration, the scheduling of administration, and other factors known to practitioners. Because of differences in patterns of bacterial accumulation in different sites as well as differences in host response, significant site-to-site and patient-to-patient variability exists. For purposes herein, the "therapeutically effective amount" of TGF-$\beta$ is an amount that is effective either to prevent, lessen the worsening of, alleviate, or cure the diseased condition.

As a general proposition, the TGF-$\beta$ is formulated and delivered to the target site at a dosage capable of establishing at the site a TGF-$\beta$ level greater than about 0.1 ng/cc. Typically, the TGF-$\beta$ concentrations range from about 0.1 ng/cc to 5 mg/cc, preferably from about 1 to 2000 ng/cc. These intra-tissue concentrations are preferably maintained, if possible, by topical application and/or sustained release.

As noted above, these suggested amounts of TGF-$\beta$ are subject to a great deal of therapeutic discretion. The key factor in selecting an appropriate dose and scheduling is the result obtained. Clinical parameters to determine an endpoint include reduction in the level of bacteria in periodontal pockets, reduction in probing depths, increase in tooth attachment levels, and increase in radiographically detectable alveolar bone height. Such measurements are well known to those clinicians and pharmacologists skilled in the art.

While the patient is being treated with TGF-$\beta$, one way to monitor the effectiveness of the treatment is to analyze the gingival crevicular fluid, as described by Kleinberg et al., Compendium, Vol. VIII, p. 624-635 (1987).

Preferably, the TGF-$\beta$ is not administered with other growth factors such as EGF or TGF-$\alpha$. However, it is within the scope hereof to combine the TGF-$\beta$ therapy with other novel or conventional therapies for the disorders in question. For example, the TGF-$\beta$ therapy may be delivered in concert with other chemotherapeutic agents as defined above or with other growth factors that influence inflammation or bone formation. It is not necessary that such cotreatment drugs be included in the TGF-$\beta$ compositions per se, although this will be convenient where such drugs are proteinaceous, such as in the case of antagonists to the activity of $\gamma$-interferon, TNF-$\alpha$, IL-1 and TNF-$\beta$ (amino acid sequence variants or neutralizing antibodies).

The TGF-$\beta$ composition is administered by any suitable means, including systemic and topical, preferably topical. Systemic includes enteral and parenteral administration, and if desired for local treatment, or rinse or spray administration. Parenteral infusions include intramuscular, intravenous, intraarterial, intraperitoneal, or subcutaneous administration or direct injection into lesions. In addition, the TGF-$\beta$ composition is suitably administered by pulse infusion, particularly with declining doses of the composition, or by continuous infusion. In another embodiment, the TGF-$\beta$ is administered in a continuous release formulation.

Such compositions are formulated appropriately for oral, topical, or parenteral administration. The active ingredient is generally combined at ambient temperature at the appropriate pH, and at the desired degree of purity, with a physiologically acceptable carrier, i.e., a carrier that is non-toxic to the patient at the dosages and concentrations employed. The carrier may take a wide variety of forms depending on the form of preparation desired for administration.

To be effective, the TGF-$\beta$ is converted to its activated form, i.e., the mature form is cleaved from its precursor using a suitable enzyme and the resultant complex is treated with acid or other appropriate agent, to activate the TGF-$\beta$. Nevertheless, TGF-$\beta$ is suitably administered in an inactive or delayed release form such as a complex of

mature TGF-$\beta$ to proTGF-$\beta$ not containing mature TGF-$\beta$ (i.e., the remaining precursor of TGF-$\beta$), to a TGF-$\beta$ binding protein or to alpha$_2$-macroglobulin. The latent form is then converted to the active form either by naturally occurring mechanisms in the local environment or by formulation with TGF-$\beta$ activating agents described above. See, e.g., Gentry et al., Mol. Cell. Biol., 8: 4162-4168 (1988); Miyazono et al., J. Biol. Chem., 263: 6407-6415- (1988); Wakefield et al., J. Biol. Chem., 263: 7646-7654 (1988); Keski-Oja et al., J. Cell Biochem. Suppl., 11A: 60 (1987); Kryceve-Martinerie et al., Int. J. Cancer, 35:553-558 (1985); Lawrence et al., Biochem. Biophys. Res. Commun., 133: 1026-1034 (1985); Lawrence et al., J. Cell Physiol., 121: 184-188 (1984). Thus, the pH of the TGF-$\beta$ composition may suitably reflect the conditions necessary for activation.

For parenteral administration, the carrier is suitably a buffer, a low molecular weight (less than about 10 residues) polypeptide, a protein, an amino acid, a carbohydrate including glucose or dextrans, a chelating agent such as EDTA, a cellulose, or other excipient. In addition, the TGF-$\beta$ composition is preferably sterile. Sterility is readily accomplished by sterile filtration through (0.2 micron) membranes. TGF-$\beta$ ordinarily will be stored as an aqueous solution, as it is highly stable to thermal and oxidative denaturation, although lyophilized formulations for reconstitution are acceptable.

For enteral use, liquid forms of the TGF-$\beta$ include suitable flavored emulsions with edible oils, such as cottonseed oil, as well as elixirs and similar pharmaceutical vehicles. Generally, where the disorder permits, one should formulate and dose the TGF-$\beta$ for site-specific delivery, where the TGF-$\beta$ is formulated into a sterile sustained-release composition suitable for injection into the tooth pocket.

Most preferably, the TGF-$\beta$ is administered by means of a dentifrice formulation, including but not limited to pastes, e.g., dental packing pastes, sticks, ointments, creams, lotions, gels, powders, e.g., dental packing powders, buccal tablets, lozenges, tooth paste, chewing gum, mouth sprays, mouth washes, permeable materials that are shaped or flexed that dissolve in an oral environment, such as semi-solid or solid dissolvable sheets or matrixes, or other oral hygienic formulations. The topical formulations are particularly preferred for the treatment of periodontitis and related diseases of the oral cavity.

If the TGF-$\beta$ formulation is to be applied topically, it is preferable to use a viscous solution such as a gel rather than a non-viscous solution. This may be accomplished, for example, by mixing the solution of TGF-$\beta$ with a gelling agent, such as a polysaccharide, preferably a water-soluble polysaccharide, such as, e.g., hyaluronic acid, starches, and cellulose derivatives, e.g., methylcellulose, hydroxyethyl cellulose, and carboxymethyl cellulose. The most preferred gelling agent is methylcellulose. The polysaccharide is generally present in a gel formulation in the range of 1-90% by weight of the gel, more preferably 1-20%. Examples of other suitable polysaccharides for this purpose, and a determination of the solubility of the polysaccharides, are found in EP 267,015, published May 11, 1988.

If formulated as a tablet, the TGF-$\beta$ is mixed with conventional tabletting ingredients such as corn starch, lactose, sucrose, sorbitol, talc, stearic acid, magnesium stearate, dicalcium phosphate, gums, or similar materials as non-toxic pharmaceutically acceptable diluents or carriers.

Tablets or pills are generally laminated or otherwise compounded to provide a dosage form affording the advantage of prolonged or delayed action of the medication. For example, the tablet or pill can comprise an inner dosage and an outer dosage component, the latter being in the form of an envelope over the former. The two components are separated by an enteric layer such as one comprising polymeric acids to resist disintegration in the stomach.

For the preparation of a sustained-release formulation, the TGF-$\beta$ is suitably incorporated into a biodegradable matrix or microcapsular particle. A suitable material for this purpose is a polylactide, although other polymers of poly ($\alpha$-hydroxycarboxylic acids), such as poly-D-(-)-3-hydroxybutyric acid (EP 133,988A), can be used. Additional biodegradable polymers include poly(lactones), poly(acetals), poly(orthoesters) or poly-(orthocarbonates). The TGF-$\beta$ is also suitably mixed with a biodegradable protein carrier such as collagen, atelocollagen, or gelatin to form a carrier matrix having sustained-release properties; the resultant mixture is then dried, and the dried material is formed into an appropriate shape, as described U.S. Pat. No. 4,774,091.

The initial consideration here must be that the carrier itself, or its degradation products, are non-toxic in the target tissue and will not further aggravate the disease. This can be determined by routine screening in animal models of the target disorder or, if such models are unavailable, in normal animals. For examples of sustained-release compositions, see U.S. Patent No. 3,773,919, EP 58,481A, U.S. Patent No. 3,887,699, EP 158,277A, Canadian Patent No. 1176565, U. Sidman et al., Biopolymers, 22:547 (1983), and R. Langer et al., Chem. Tech., 12:98 (1982).

Controlled delivery of TGF-$\beta$ to a periodontal pocket also is suitably accomplished using permeable hollow cellulose acetate fibers with the

TGF-$\beta$ and placed in the pockets and removed 24 hours later or left for longer periods of time (U.S. Pat. No. 4,175,326). Also, acrylic resin strips or cast films can be impregnated with TGF-$\beta$ and applied to the affected site. In addition, narrow dialysis tubing can be filled with a TGF-$\beta$ solution and placed so as to deliver TGF-$\beta$ to the appropriate site.

In addition, local delivery of TGF-$\beta$ to periodontal lesions via non-degradable hollow fibers placed into the lesion with dental instruments is applicable herein. See Lindhe et al., J. Clin. Periodontol., 6: 141 (1979).

Other methods include long-acting capsules or tablets held in the mouth (U.S. Pat. No. 3,911,099); buccal implants for releasing drugs into the saliva (U.S. Pat. No. 4,020,558); topically applied gels (U.S. Pat. No. 3,679,360); topically applied drug-containing bandages (U.S. Pat. No. 3,339,546); a drug-containing plastic hardenable mass (U.S. Pat. No. 3,964,164); a medicated periodontal dressing (U.S. Pat. No. 3,219,527); a topical dressing composed of a finely divided particulate carrier and suspended medicinal agents (U.S. Pat. No. 3,698,392); a bandage for covering moist mucosal surfaces (U.S. Pat. No. 3,339,546); a microencapsulated liquid droplet formulation for topical application to the gums of animals (U.S. Pat. No. 4,329,333); foam-film devices containing the drug (U.S. Pat. No. 3,844,286); impregnated or drug-releasing forms of dental flows (U.S. Pat. No. 3,417,179); a soft polysaccharide film or sheet optionally with a plasticizer and sterilized by heating or by ethylene oxide or radiation treatment (U.S. Pat. No. 4,569,837); a self-gelling liquid composition for topical application with ethyl orthosilicate (U.S. Pat. No. 4,454,110); an ethylcellulose film cut to the approximate dimension of a pocket (U.S. Pat. No. 4,568,535); solid absorbable fibers of polyglycolic acid with drugs incorporated therein (U.S. Pat. No. 3,991,766); a time-release microshape encapsulating TGF-$\beta$ for insertion into a vacuous cavity (U.S. Pat. No. 4,685,883); an absorbable putty-like matrix (U.S. Pat. No. 4,650,665); or a polymeric material having the drug impregnated thereon (U.S. Pat. No. 4,764,377).

Another method of delivery is to use supragingival irrigation, e.g., by pumping a dilute aqueous solution of TGF-$\beta$ through an orifice to provide a jet stream and a pulsation penetrating directly into periodontal pockets and applying the jet stream along the margins between the teeth and gums to cleanse the tissues and provide a residue of TGF-$\beta$, as described in U.S. Pat. No. 4,683,133. In addition, an oral lavage of TGF-$\beta$ is suitably applied to the periodontium and teeth by a rinsing action for one or more periods daily of about 30 seconds followed by expectoration. Alternatively, the oral lavage is applied to the gums and teeth by irrigation for at least 2 minutes.

The invention herein is useful for patients who lack the requisite degree of motivation or manual dexterity to remove plaque mechanically; thus, chemical control of the plaque using TGF-$\beta$ is employed rather than mechanical removal. In addition, the composition herein is useful to treat patients at the post-operative period. After surgery it is painful to maintain oral hygiene. Therefore, for example, a 10 ml solution of a 0.2 or 0.1% solution of TGF-$\beta$ is given as a mouth rinse for two one-minute periods daily until healing occurs. Alternatively, after treatment with TGF-$\beta$, another chemotherapeutic agent, such as chlorhexidine, is useful as a mouth rinse.

The invention will be more fully understood by reference to the following examples.

EXAMPLE 1

The TGF-$\beta$ used herein is from one of several sources: (1) porcine platelet-derived TGF-$\beta_1$ at 96 percent purity purchased in a lyophilized form and suspended in 4 mM HCl and phosphate buffered saline (PBS) solution prior to use, (2) TGF-$\beta_1$, prepared by recombinant means as described by EP 200,341, supra, (3) TGF-$\beta_2$, prepared as described by U.S. Pat. No. 4,774,322, supra, or (4) TGF-$\beta_3$, prepared by recombinant means as described by EP 267,463, supra (see Fig. 29 and accompanying text of EP 267,463).

The TGF-$\beta$ from any of these sources is recovered and formulated at 1 - 10 ng/g with ethylcellulose, as described in U.S. Pat. No. 4,568,535. The resulting film is 8 mm in length, approximately the dimension of a periodontal pocket.

Human adults having active periodontitis with periodontal pockets of 6 mm are chosen for the study. The periodontal pocket is the space between gum and tooth in periodontal disease that does not exist in healthy gums. This space offers a site for bacterial infection and inflammation that destroys the surrounding gum and osseous support for the tooth, and eventually causes the tooth to be lost.

The patients are assigned to one of two groups. The first group of patients are treated by placing the ethylcellulose films containing TGF-$\beta$ in the deep periodontal pockets. The second group of patients have placed within their deep periodontal pockets ethylcellulose films without TGF-$\beta$. The 2 mm of film that extends out of each pocket is secured to the tooth by using a cyanoacrylate glue. The film that is designed to be placed in the pocket is contoured by the clinician so as to be placed below the gingival margin and to occupy most of the space of the pocket.

The pocket depth is measured prior to and after placement of the films in the pockets using a University of Michigan periodontal probe to measure any difference in pocket depth from the beginning of the study until its conclusion. Pocket depth measurements are made on four sides of each tooth. All four readings for each tooth are then added together for all of the teeth of a given patient, and the result is divided by the total number of the patient's teeth to provide an average score per tooth for the sum of the pocket depths on the four sides. A decrease in average pocket depth thus represents an improvement in the patient's condition and a positive response to the treatment being employed.

The films are left in the pockets for two weeks. After this time, the pocket depths are analyzed. The TGF-$\beta$ is shown to effect a reduction in pocket depth of the TGF-$\beta$-treated patients versus that of the control patients.

EXAMPLE 2

An aqueous-based gel of methylcellulose (Methocel A4M from Dow) is prepared by mixing it in water at a concentration of 20% by weight to give a viscosity useful for a topical skin application. The TGF-$\beta_1$ recombinantly produced as described in Example 1 is mixed with the gel to provide a concentration of 1 mg/ml.

The resulting gel is applied to periodontal pockets of five adult human patients with advanced periodontal diseases. A control group of five similar patients is not so treated. The results indicate that the probing depths of the pockets of the treated patients measured as described in Example 1 are decreased relative to that of the control group.

EXAMPLE 3

Braided silk 4-0 ligatures were soaked in TGF-$\beta_1$ recombinantly produced as described in Example 1 and dried before use. The impregnated silk contained about 200 ng/cm of TGF-$\beta_1$. Release experiments performed in 10 ml PBS and 0.5% bovine albumin indicated that 32%, 81%, and 100% of the active protein were released after 10, 60, and 180 min., respectively.

Two adult female Macaca fascicularis (non-human primates) had ligatures tied around their second premolar, and first and second molar in one posterior sextant as a periodontitis control. TGF-$\beta$-

treated ligatures were similarly placed in two sextants, and the fourth sextant was untreated as a gingivitis control. Weekly, for four weeks, the ligatures were replaced. The TGF-$\beta_1$ was found to have a significant effect on pocket depth when analyzed by tooth site, but not by animal.

The invention can thus provide for a dental composition for the treatment of periodontitis which is effective in both supragingival and subgingival treatment.

The composition is also capable of restoring the regrowth of the periodontium, including alveolar bone and of the peridontal connective tissue, and reducing the release of additional collagenase.

## Claims

1. A dental composition for the use in administration to the gum of a mammal, comprising a physiologically effective amount of transforming growth factor-$\beta$ (TGF-$\beta$) in a pharmaceutically acceptable carrier.

2. A composition according to claim 1 wherein the composition is in the form of a paste, an ointment, a cream, a lotion, a gel, a powder, a buccal tablet, a lozenge, toothpaste, chewing gum, a mouth spray, a mouth wash, guar gum, or a perneable material that is shaped or flexes to fit around the teeth or gums of the mammal and is capable of dissolving upon placement in an oral environment.

3. A dental composition according to claim 1 or claim 2 wherein the TGF-$\beta$ is TGF-$\beta_1$, TGF-$\beta_2$ or TGF-$\beta_3$ and the mammal is human.

4. A dental composition according to any one of claims 1 to 3 wherein the composition further comprises an effective amount of a chemotherapeutic agent.

5. A composition according to any of claims 1 to 4 for topical application of the composition to the site of a diseased gum.

6. A dental composition according to claim 5 wherein the composition is in the form of an ointment, a cream, or a permeable sheet or matrix that flexes to fit around the teeth or gums of the mammal and is capable of dissolving upon placement in an oral environment.

7. A dental composition according to claim 5 or claim 6 which enables the delivery of 0.1 ng/cc to 5 mg/cc of TGF-$\beta$ to the site.

8. A permeable material for use in the delivery of a dental composition that is shaped or flexes to fit around the teeth or gums of a mammal, which material is treated with transforming growth factor-$\beta$ (TGF-$\beta$ ) and is capable of dissolving upon placement in an oral environment.

9. A material according to claim 8 that is a membrane, strip, film, fibers, sheet, bandage, plastic hardenable mass, periodontal dressing, microencapsulated liquid droplet formulation, or polymeric matrix.

10. A material according to claim 8 or claim 9 that is a polymeric matrix composed of collagen, glycolic acid polymers, methacrylate polymers, or polylactides and that is impregnated with the TGF-$\beta$.

11. The use of TGF-$\beta$ for the preparation of a dental composition for the treatment or prophylaxis of gingivitis or periodontitis of a gum of a mammal.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| X | AGENTS AND ACTIONS vol. 22, no. 3/4, December 1987, BASEL pages 251 - 254; HAYWARD M. et al: "Mechanisms of bone loss: rheumatoïd arthritis, periodontal disease and osteoporosis" * the whole document * | 1-11 | A61K37/02 A61L15/03 A61L15/32 |
| X | JOURNAL OF DENTAL RESEARCH vol. 66, March 1987, USA page 99 RUSSEL R.G.G.: "The cellular control of bone formation and resorption." * line s38 * | 1-11 | |
| X | JOURNAL OF DENTAL RESEARCH· vol. 67, March 1988, USA page 185 COTUGNO K. et al: "Transforming growth factor beta is a chemoattractant for periodontal ligament cells." * line 581 * | 1-11 | |
| X,P | JOURNAL OF DENTAL RESEARCH vol. 68, June 1989, USA page 933 MATSUE M. et al: "Stimulation of collagen production and initial wound healing by TGF-beta." * line 536 * | 1-11 | **TECHNICAL FIELDS SEARCHED (Int. Cl.5 )** A61K A61L |
| X | EP-A-105014 (THE UNITED STATES OF AMERICA) * page 25, line 21 * | 1-11 | |
| A | EP-A-269408 (GENENTECH) * the whole document * | 1-11 | |
| A | EP-A-213776 (COLLAGEN CORPORATION) * the whole document * | 1-11 | |

The present search report has been drawn up for all claims

| Place of search | Date of completion of the search | Examiner |
|---|---|---|
| BERLIN | 09 FEBRUARY 1990 | AVEDEKIAN P.F. |